# EUROPEAN PATENT APPLICATION

(11) **EP 1 801 110 A1**
(43) Date of publication of application: **27.06.2007**
(21) Application number: 05028252.4
(22) Date of filing: 22.12.2005
(51) Int. Cl.: C07D 401/12, A61K 31/4439, A61P 1/04

(54) **Esomeprazole arginine salt**

(71) Applicant: KRKA, tovarna zdravil, d.d., Novo mesto, 8501 Novo mesto (SI)
(72) Inventor: Kotar-Jordan, Berta, SLO-8311 Kostanjevica na Krki (SI); Pisek, Robert, SLO-1000 Ljubljana (SI); Zajc, Natalija, SLO-8340 Crnomelj (SI); Vajs, Anamarija, SLO-8000 Novo mesto (SI)
(74) Representative: UEXKÜLL & STOLBERG

(57) **Abstract**

The esomeprazole arginine salt as well as processes for its preparation and pharmaceutical compositions containing this salt are described.

## Description

The present invention relates to esomeprazole arginine salt as well as processes for its preparation and pharmaceutical compositions containing this salt.

Esomeprazole is the (S) (-) enantiomer of omeprazole, a sulfoxide which has an asymmetric center at the sulfur atom and exists in form of different enantiomers. The chemical name of omeprazole is 5-methoxy-2-[[(4-methoxy-3,5-dimethyl-2-pyridinyl)-methyl]sulphinyl]-1H-benzimidazole.

Omeprazole is in-vivo transformed into an effective inhibitor of gastric acid secretion and is therefore useful as an anti-ulcer agent or for preventing and/or treating gastric acid related disorders and gastrointestinal inflammatory diseases in mammals.

In man, for example, omeprazole may be used to prevent and/or treat gastritis, gastric ulcer and duodenal ulcer.

Omeprazole, and its therapeutically acceptable alkaline salts are disclosed in EP 5 129 and EP 124 95, respectively.

DE 4035455 discloses the separation of the enantiomers of omeprazole using a diastereomeric ether.

WO 94/27988 discloses certain salts, namely Na+, Mg2+, Li+, K+, Ca2+ and N(R)₄⁺ salts, wherein R is an alkyl with 1-4 carbon atoms, of the single enantiomers of omeprazole and their preparation. These compounds are said to have improved pharmacokinetic properties which give an improved therapeutic profile, such as a lower degree of variation between individuals taking the compound.

Various other salts of esomeprazole have also become known.

WO 98/54171 describes magnesium esomeprazole trihydrate; WO 00/44744 describes specific polymorphs of the potassium salt of esomeprazole; WO 2003/74514 discloses some primary amine salts of esomeprazole; WO 2004/037253 describes certain alkali metal or amine salts of esomeprazole; WO 2004/99182 and WO 2004/99181 disclose zinc and barium salts of esomeprazole, respectively; WO2005023796 discloses adamantan ammonium salts of omeprazole and esomeprazole; and WO2005023797 describes the 1-cyclohexyl ethyl ammonium salt of inter alia esomeprazole.

Finally, EP 1 018 340 describes amino acid salts compounds of benzimidazole derivatives and their inclusion complexes with cyclodextrins. The amorphous L-arginine omeprazole salt is described which is produced by spray drying. However, there is no mentioning of esomeprazole arginine salt.

Even though several salt of esomeprazole are know, there is, however, still a need for further salts of esomeprazole which show a high stability and advantageous properties when included in pharmaceutical compositions.

The present invention meets this need by providing the esomeprazole arginine salt according to claims 1 to 3. The invention also relates to the process for preparing this salt according to claims 4 to 7 and pharmaceutical compositions comprising this salt according to claims 8 to 13.

First of all, the invention provides the esomeprazole arginine salt. It is a salt of the know drug esomeprazole with the amino acid arginine and could also be referred to as arginine esomeprazole salt. Surprisingly, this salt shows a very high stability and is therefore very suitable to be included in pharmaceutical compositions.

It is preferred that in this salt the molar ratio between esomeprazole and arginine is about 1:1 and that arginine is L-arginine.

Further is preferred that the salt is in amorphous form as this may facilitate its processing into pharmaceutical compositions.

The salt has been characterized by its FT-IR spectrum, X-ray powder diffraction pattern and ¹H-NMR spectrum and analysed in accordance with the article »Enantioselective Analysis of Omeprazole in Pharmaceutical Formulations by Chiral High-Performance Liquid Chromatography and Capillary Electrophoresis« (J. Braz. Chem. Soc., Vol. 15, No. 2, 318-323, 2004).

FT-IR spectra of KBr discs including the salt according to the invention were recorded over a wave number range of 4000 - 400 cm⁻¹ on a Perkin Elmer Spectrum GX FT-IR spectrometer at a resolution of 4 cm⁻¹.

X-ray powder diffraction patterns were obtained by using a Phillips PW3040/60 X'Pert PRO powder diffractometer; CuKα radiation 1.541874Å, 3 <2θ<31°.

The ¹H-NMR spectrum has been recorded by means of a Varian UNITY+ 300 spectrometer at 300 MHz using DMSO-d6 and tetramethyl silane as internal standard.

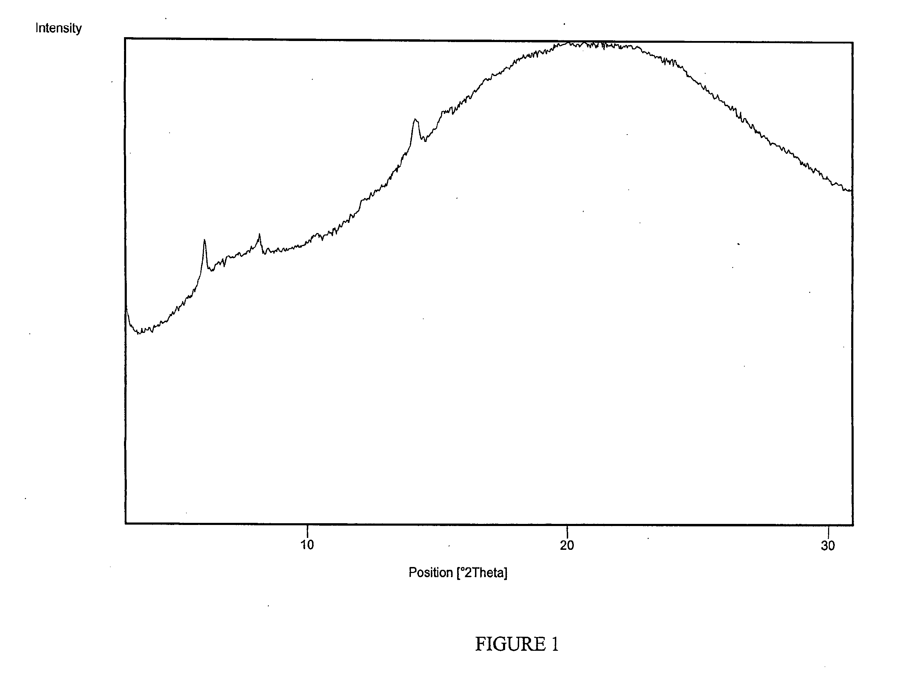

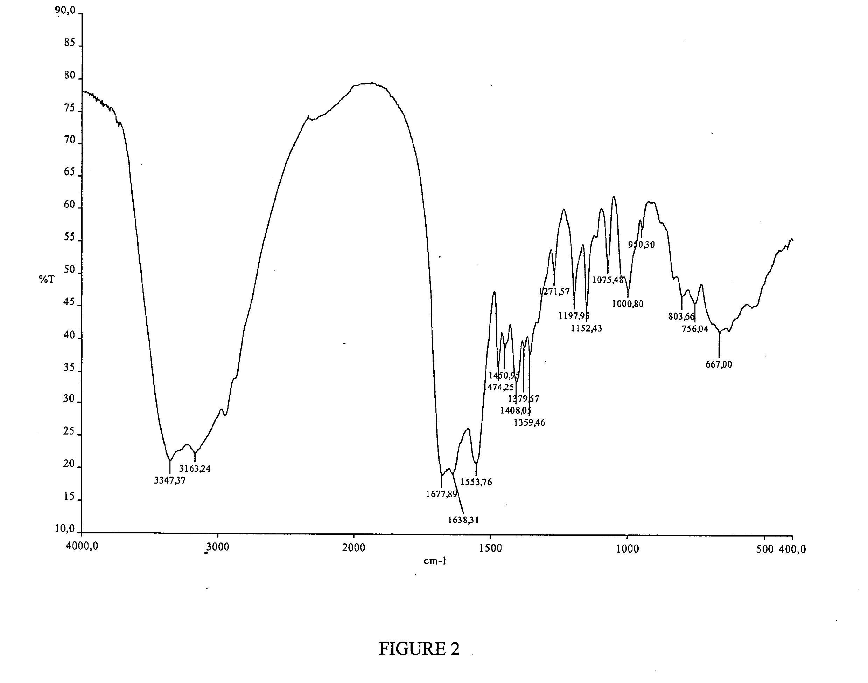

Figure 1 shows the X-ray powder diffractogram and Figure 2 the FT-IR spectrum of amorphous esomeprazole arginine salt.

The salt according to the invention is preferably prepared by a process, wherein esomeprazole and arginine are reacted in a solvent and the solvent is removed.

The solvent is in particular selected from water, an alcohol or a mixture thereof. The preferred alcohol is methanol, ethanol, 2-propanol, n-propanol or butanol.

In a preferred embodiment, the process is carried out until all esomeprazole was dissolved or the pH value of the reaction mixture remained constant.

The molar ratio between the esomprazole and the arginine can vary, but is preferably about 1:1.

Usually the esomeprazole is dissolved in the selected solvent and then arginine is added. After completion of the reaction the solvent is removed by for example distillation, distillation under vacuum, evaporation, spray drying, and freeze drying.

The esomeprazole used in the process according to the invention can be prepared according to any desired method, many of which are known to those of ordinary skill in the art. Examples of suitable methods to manufacture esomeprazole are given by EP 5 129, EP 124 495, EP 533 752, DE 40 35 455, EP 652 872, EP 707 580, EP 773 940, EP 795 024, EP 836 601, EP 897 386, EP 946 547, EP 984 957, EP 964 859, EP 1 004 305, EP 1 095 037, EP 1 104 417, EP 1 230 237, EP 1 409 478, EP 1 458 709, WO 03/08940, EP 1 375 497, WO 2004/2982, WO 2004/52882, WO 2004/87702, EP 1 375 497, WO 2005/54228, and WO 2005/116011.

The invention also relates to pharmaceutical compositions which comprise the salt according to the invention. These compositions may be used for the treatment of gastric-acid related diseases by inhibition of gastric acid secretion.

The pharmaceutical compositions according to the invention are preferably in the form of pellets or tablets, in particular those which have an enteric coating layer. The pellets can be filled into hard gelatine capsules or sachets which hence also represent preferred embodiments of the composition. The tables can be prepared from the pellets or from a powder mixture.

The pellets preferably comprise
(a) a core including the esomeprazole arginine salt according to the invention,
(b) optionally at least one separating layer,
(c) at least one enteric layer, and
(d) optionally at least one over-coating layer.

The tablets preferably comprise
(a) a core including the esomeprazole arginine salt according to the invention,
(b) optionally at least one separating layer, and
(c) at least one enteric layer.

In a preferred embodiment of both, the pellets and the tablets, the salt is included in the core in form of a layer covering the core.

The invention also relates to multiple-unit tablets which are obtainable by compressing a mixture of the pellets with tablet excipients.

Further preferred embodiments of pellets and tablets according to the invention and preferred ways of producing them are described below.

### A. Pellets

Preferred pellets comprises a pellet core, optionally a separating coating, an enteric coating and optionally an over-coating as follows.

### 1. Core

The core is prepared from powders comprising esomeprazole arginine and at least one pharmaceutically acceptable excipient by extrusion-spheronization or direct pelletization in a high-shear mixer or a rotor granulator.

Alternatively, the core can also be formed by applying a layer containing esomeprazole arginine to an inert bead. The most preferred bead is one prepared from starch and sucrose, even though beads of other pharmaceutically acceptable excipients may be used, such as microcrystalline cellulose, vegetable gums, waxes and the like. The size of the beads may vary between approximately 0.1 and 2 mm.

A convenient manner of coating the beads with esomeprazole arginine salt is the "powder layering" process in centrifugal equipment, i.e. rotor fluid bed equipment (Glatt Rotor Granulator), or a coating pan, i.e. a conventional coating pan (Pellegrini Coating Pan, GS Coating System). The inert beads are moistened with a solution of binder, and then the active substance together with other excipients is added as a powder and the layered pellets are dried in the same equipment in which the coating is performed or other specialized equipment for drying, such as a drying chamber with or without vacuum, is used.

The layer with esomeprazole arginine salt can also be formed in the "suspension layering" or "solution layering" process by spraying the esomeprazole arginine suspension or solution onto the inert cores in a fluid bed coater granulator. Organic solvents or water can be used during this process.

The core, and in particular the layer with the active ingredient, comprises, besides esomeprazole arginine salt, at least one excipient selected from stabilizers, fillers, disintegrating agents, wetting agents, binders or other pharmaceutically acceptable ingredients, alone or in mixtures.

The stabilizer is preferably chosen among substances, such as sodium, potassium, calcium, magnesium and aluminium salts of phosphoric acid, carbonic acid, citric acid or other suitable weak inorganic or organic acids, aluminium hydroxide/sodium bicarbonate coprecipitate, aluminium, calcium and magnesium hydroxides, magnesium oxide or composite substances, such as Al₂O₃·6MgO·CO₂·12H₂O, (Mg₆Al₂(OH)₁₆CO₃·4H₂O), MgO·Al₂O₃·2SiO₂·nH₂O or similar compounds, sodium lauryl sulphate, alkaline reacting amino acids and their salts or other similar, pharmaceutically acceptable pH-buffering substances.

Furthermore, the filler is preferably selected from the group consisting of microcrystalline cellulose, sucrose, starch, lactose, mannitol, sorbitol and mixtures thereof.

The disintegrating agent is preferably selected from the group consisting of starch, starch derivatives such as sodium starch glycolate or pregelatinized starch, low-substituted hydroxypropyl cellulose, crospovidone, croscarmelose sodium and mixtures thereof.

The wetting agent is preferably selected from the group consisting of sodium dodecyl sulphate, polyoxyethylene sorbitan fatty acid esters, poloxamers and mixtures thereof.

The binder is preferably selected from the group consisting of polyvinylpyrrolidone, hydroxypropyl cellulose, hydroxypropyl methylcellulose, hydroxyethyl cellulose, methylcellulose and mixtures thereof.

### 2. Separating layer

Before applying the enteric coating layer onto the cores present in form of individual pellets, said pellets may optionally be covered with one or more separating layers comprising pharmaceutical excipients optionally including alkaline compounds, such as for instance pH-buffering compounds. This layer/these layers separate(s) the core material from the outer enteric coating layer. The separating layer can be applied to the core by a coating or layering process in a suitable equipment such as a coating pan, coating granulator or in a fluid bed apparatus using water and/or organic solvents for the coating process. As an alternative the separating layer can be applied to the core by using the powder coating technique. The materials for separating layers are pharmaceutically acceptable compounds, such as sugar, polyethylene glycol, polyvinylpyrrolidone, polyvinyl alcohol, polyvinyl acetate, hydroxypropyl cellulose, methylcellulose, ethylcellulose, hydroxypropyl methylcellulose, carboxymethylcellulose sodium and others, used alone or in mixtures. Additives such as plasticizers, colorants, pigments, fillers, anti-tacking agents and anti-static agents, such as for instance magnesium stearate, titanium dioxide, talc and other additives may also be included in the separating layer.

The separating layer may serve as a diffusion barrier and may act as a pH-buffering zone. The pH-buffering properties of the separating layer can be further strengthened by introducing into the layer substances chosen from a group of compounds usually used in antacid formulations, such as magnesium oxide, hydroxide or carbonate, aluminium or calcium hydroxide, carbonate or silicate, composite aluminium/magnesium compounds, such as Al₂O₃·6MgO·CO₂·12H₂O, (Mg₆Al₂(OH)₁₆CO₃·4H₂O), MgO·Al₂O₃·2SiO₂·nH₂O, aluminium hydroxide/sodium bicarbonate coprecipitates or similar compounds, or other pharmaceutically acceptable pH-buffering compounds, such as the sodium, potassium, calcium, magnesium and aluminium salts of phosphoric, carbonic, citric or other suitable, week, inorganic or organic acids, or suitable organic bases, including basic amino acids and salts thereof. Talc or other compounds may be added to increase the thickness of the layer and thereby strengthen the diffusion barrier.

The optionally applied separating layer is not essential for the compositions according to the invention. However, the separating layer may improve the chemical stability of the active substance and/or the physical properties of the final dosage form.

The separating layer may also protect the enteric coating layer towards cracking during a compaction process. For this purpose the separating layer preferably contains pharmaceutically acceptable plasticizers to obtain the desired mechanical properties, such as flexibility and hardness. Such plasticizers are for example triacetin, citric acid esters, phthalic acid esters, dibutyl sebacate, dimethyl polysiloxan, cetyl alcohol, stearyl alcohol, polyethylene glycols, propylenglycole, polysorbates or other plasticizers. The mechanical properties, i.e. flexibility and hardness of the separating layer are adjusted so that the acid resistance of the pellets covered with enteric coating layer does not decrease significantly during the compression of pellets into tablets. The amount of plasticizer is usually 10-50 % by weight of the separating layer forming material. Additives such as dispersants, colorants, pigments, polymers, anti-tacking agent and antifoaming agents may also be included into the separating layer. The maximum thickness of the applied separating layer is normally only limited by processing conditions.

### 3. Enteric layer

One or more enteric coated layers are applied onto the core or onto the core covered with separating layer(s) by using a suitable coating technique. The enteric coating layer material may be dispersed or dissolved in either water or in suitable organic solvents and is preferably comprising a polymer. As enteric coating layer polymers one or more, separately or in combination, of the following can be used: solutions or dispersions of methacrylic acid copolymers, cellulose acetate phthalate, hydroxypropyl methylcellulose phthalate, hydroxypropyl methylcellulose acetate succinate, polyvinyl acetate phthalate, cellulose acetate trimellitate, carboxymethylethylcellulose, shellac or other suitable enteric coating layer polymer(s).

The enteric coating layers can also contain pharmaceutically acceptable plasticizers to obtain the desired mechanical properties, such as flexibility and hardness. Such plasticizers are for example those mentioned above under 2. Separating layer.
The amount of plasticizer is optimized for each enteric coating layer formula, in relation to the selected enteric coating layer polymer(s), selected plasticizer(s) and the applied amount of said polymer(s), in such a way that the mechanical properties, i.e. flexibility and hardness of the enteric coating layer(s) are adjusted so that the acid resistance of the pellets covered with enteric coating layer(s) does not decrease significantly during a compression of the pellets into tablets. The amount of plasticizer is usually 10-50 % by weight of the enteric coating layer polymer(s). Additives such as dispersants, colorants, pigments, polymers, anti-tacking agent and antifoaming agents may also be included into the enteric coating layer(s). Other compounds may be added to increase film thickness and to decrease diffusion of acidic gastric juices into the acid susceptible material.

To protect an acidic susceptible substance in the form of esomeprazole arginine salt and to obtain an acceptable acid resistance of the multi-unit tableted dosage form, the enteric coating layer(s) usually have a thickness of about at least 10 µm, preferably more than 20 µm. The maximum thickness of the applied enteric coating layer(s) is normally only limited by processing conditions.

### 4. Over-coating layer

The enteric coated pellets can optionally be covered with one or more over-coating layers. The over-coating layer can be applied to the enteric coating layered pellets by coating or layering procedures in a suitable equipment, such as a coating pan, a coating granulator or in a fluidized bed apparatus using water and/or organic solvents for the coating process. The materials for the over-coating layer are chosen among pharmaceutically acceptable compounds, such as sugar, polyethylene glycol, polyvinylpyrrolidone, polyvinyl alcohol, polyvinyl acetate, hydroxypropyl cellulose, methylcellulose, ethylcellulose, hydroxypropyl methylcellulose, microcrystalline cellulose, carboxymethylcellulose sodium and others, used alone or in mixtures. Additives such as plasticizers, colorants, pigments, fillers, anti-tacking agents and anti-static agents, such as magnesium stearate, titanium dioxide, talc and other additives may also be included into the over-coating layer.

The over-coating layer may further prevent potential agglomeration of enteric coating layered pellets and further enhance a subsequent tableting process. The over-coating layer may also protect the enteric coating layer towards cracking during a compaction process. For this purpose the over-coating layer contains pharmaceutically acceptable plasticizers to obtain the desired mechanical properties, such as flexibility and hardness. Such plasticizers are for example triacetin, citric acid esters, phthalic acid esters, dibutyl sebacate, dimethyl polysiloxan, cetyl alcohol, stearyl alcohol, polyethylene glycols, propylenglycole, polysorbates or other plasticizers. The mechanical properties, i.e. flexibility and hardness of the over-coating layer are adjusted so that the acid resistance of the pellets covered with enteric coating layer does not decrease significantly during a compression of the pellets into tablets. The amount of plasticizer is usually 10-50 % by weight of the over-coating layer forming material. Additives such as dispersants, colorants, pigments, polymers, anti-tacking agent and antifoaming agents may also be included into the over-coating layer. The maximum thickness of the applied over-coating layer is normally only limited by processing conditions.

### B. Tablets, prepared from pellets (Multiple unit tablets)

The pellets according to the invention can be used for the preparation of multiple unit tablets.

For this purpose the enteric coated pellets with or without an over-coating layer are mixed with tablet excipients, such as fillers, binders, disintegrating agents, lubricants and other pharmaceutically acceptable additives, and compressed into tablets. The compressed tablet is optionally covered with a film forming agent to obtain a smooth surface of the tablet and further enhance the stability of the tablet during packaging and transport.

### C. Tablets, prepared from powder mixtures

In another preferred embodiment, the pharmaceutical composition is in form of tablets which comprise a tablet core, optionally a separating coating , and an enteric coating.

The preferred composition and the preferred manner for producing such tablets is given hereinafter.

### 1. Tablet cores

The tablet cores are prepared from powder mixtures comprising the active substance and at least one pharmaceutically acceptable excipient by using the process of direct compression. Alternatively, said tablet cores can also be formed in the process of wet- or dry-granulation. The tablet cores comprise, besides esomeprazole arginine salt, at least one excipient selected from stabilizers, fillers, disintegrating agents, wetting agents, binders or other pharmaceutically acceptable ingredients, alone or in mixtures.

The stabilizer is preferably chosen among substances such as sodium, potassium, calcium, magnesium and aluminium salts of phosphoric acid, carbonic acid, citric acid or other suitable weak inorganic or organic acids, aluminium hydroxide/sodium bicarbonate coprecipitates, aluminium, calcium and magnesium hydroxides, magnesium oxide or composite substances such as Al₂O₃·6MgO·CO₂·12H₂O, (Mg₆Al₂(OH)₁₆CO₃·4H₂O), MgO·Al₂O₃·2SiO₂·nH₂O or similar compounds, sodium lauryl sulphate, alkaline reacting amino acids and their salts or other similar, pharmaceutically acceptable pH-buffering substances.

Furthermore, the filler is preferably selected from the group consisting of microcrystalline cellulose, sucrose, starch, lactose, mannitol, sorbitol and mixtures thereof.

The disintegrating agent is preferably selected from the group consisting of starch, starch derivatives such as sodium starch glycolate or pregelatinized starch, low-substituted hydroxypropyl cellulose, crospovidone, croscarmelose sodium and mixtures thereof.

The wetting agent is preferably selected from the group consisting of sodium dodecyl sulphate, polyoxyethylene sorbitan fatty acid esters, poloxamers and mixtures thereof.

The binder is preferably selected from the group consisting of polyvinylpyrrolidone, hydroxypropyl cellulose, hydroxypropyl methylcellulose, hydroxyethyl cellulose, methylcellulose and mixtures thereof.

### 2. Separating layer

Before applying the enteric coating layer onto the tablet cores, said tablet cores may optionally be covered with one or more separating layers comprising pharmaceutical excipients optionally including alkaline compounds, such as pH-buffering compounds. This layer/these layers separate(s) the core material from the outer enteric coating layer. The separating layer can be applied to the core by a coating or layering process in a suitable equipment, such as a coating pan, a coating granulator or in a fluid bed apparatus using water and/or organic solvents for the coating process. As an alternative the separating layer can be applied to the tablet core by using a suitable coating technique. The materials for separating layers are pharmaceutically acceptable compounds, such as sugar, polyethylene glycol, polyvinylpyrrolidone, polyvinyl alcohol, polyvinyl acetate, hydroxypropyl cellulose, methylcellulose, ethylcellulose, hydroxypropyl methylcellulose, carboxymethylcellulose sodium and others, used alone or in mixtures. Additives such as plasticizers, colorants, pigments, fillers, anti-tacking agents and anti-static agents, such as magnesium stearate, titanium dioxide, talc and other additives may also be included into the separating layer.

The separating layer may serve as a diffusion barrier and may act as a pH-buffering zone. The pH-buffering properties of the separating layer can be further strengthened by introducing into the layer substances chosen from a group of compounds usually used in antacid formulations, such as magnesium oxide, hydroxide or carbonate, aluminium or calcium hydroxide, carbonate or silicate, composite aluminium/magnesium compounds, such as Al₂O₃·6MgO·CO₂·12H₂O, (Mg₆Al₂(OH)₁₆CO₃·4H₂O), MgO·Al₂O₃·2SiO₂·nH₂O, aluminium hydroxide/sodium bicarbonate coprecipitates or similar compounds, or other pharmaceutically acceptable pH-buffering compounds, such a the sodium, potassium, calcium, magnesium and aluminium salts of phosphoric, carbonic, citric or other suitable, week, inorganic or organic adds, or suitable organic bases, including basic amino acids and salts thereof. Talc or other compounds may be added to increase the thickness of the layer and thereby strengthen the diffusion barrier. The optionally applied separating layer is not essential for the tablets according to the invention. However, the separating layer may improve the chemical stability of the active substance and/or the physical properties of the tablet.

### 3. Enteric layer

One or more enteric coating layers are applied onto the tablet cores or onto the tablet cores covered with separating layer(s) by using a suitable coating technique. The enteric coating layer material may be dispersed or dissolved in either water or in suitable organic solvents and is preferably comprising a polymer. As enteric coating layer polymers one or more, separately or in combination, of the following can be used: solutions or dispersions of methacrylic acid copolymers, cellulose acetate phthalate, hydroxypropyl methylcellulose phthalate, hydroxypropyl methylcellulose acetate succinate, polyvinyl acetate phthalate, cellulose acetate trimellitate, carboxymethylethylcellulose, shellac or other suitable enteric coating layer polymer(s).

The enteric coating layers may contain pharmaceutically acceptable plasticizers to obtain the desired mechanical properties, such as flexibility and hardness of the enteric coating layers. Such plasticizers are for the instance, but not restricted to, triacetin, citric acid esters, phthalic acid esters, dibutyl sebacate, dimethyl polysiloxan, cetyl alcohol, stearyl alcohol, polyethylene glycols, propylenglycole, polysorbates or other plasticizers. The amount of plasticizer is optimized for each enteric coating layer formula, in relation to selected enteric coating layer polymer(s), selected plasticizer(s) and the applied amount of said polymer(s), in such a way that the mechanical properties, i.e. flexibility and hardness of the enteric coating layer(s) are adjusted. The amount of plasticizer is usually 10-50 % by weight of the enteric coating layer polymer(s). Additives such as dispersants, colorants, pigments, polymers, anti-tacking agent and antifoaming agents may also be included into the enteric coating layer(s). Other compounds may be added to increase film thickness and to decrease diffusion of acidic gastric juices into the acid susceptible material.

To protect an acidic susceptible substance in the form of esomeprazole arginine salt and to obtain an acceptable acid resistance of the tableted dosage form, the enteric coating layer(s) constitutes a thickness of about at least 10 µm, preferably more than 20 µm. The maximum thickness of the applied enteric coating layer(s) is normally only limited by processing conditions.

The invention is in the following further illustrated by means of examples.

### Examples

### Example 1 - Preparation of esomeprazole arginine salt (5-methoxy-2-[(S)-[(4-methoxy-3,5-dimethyl-2-pyridinyl)methyl]sulphinyl]-1H-benzimidazole arginine)

3.0 g (0.017 mole) of L-arginine were dissolved in a mixture of 110 ml of water and 60 ml of methanol. 6.0 g (0.017 mole) of esomeprazole were added and it was stirred until a solution was obtained. Any undissolved particles are filtered off to get a clear solution. The product was isolated by lyophilization. The obtained freeze dried product was further dried under reduced pressure at room temperature and subsequently at 30-35° for 3 hours. The amorphous salt of esomeprazole arginine was produced with 100 % yield and characterized by the following analyses:
¹H-NMR (300 MHz, DMSO-d₆): 1.5 (m, 4H, CH₂-arginine), 2.18 (s, 3H, CH₃), 2.20 (s, 3H, CH₃), 3.07 (m, 2H, CH₂-arginine), 3.15 (m, 1H, CH-arginine), 3.68 (s, 3H, OCH₃), 3.77 (s, 3H, OCH₃), 4.55 (d, J_{HA,B}= 13.4Hz, 1H, H_{A}), 4.77 (d, J_{HA,B}= 13.4Hz, 1H, H_{B}), 6.77 (dd, J_{H6,7}=8.8Hz, J_{H4.7}=2.4Hz, 1H, H₇), 7.05 (d, J_{H4,7}=2.4Hz, 1H, H₄), 7.45 (d, J_{H6,7}=8.8Hz, 1H, H₆), 8.19 (s, 1H, H₆,)
HPLC (Chiral purity) = 100.0%
Moisture content (KF) = 0.84% (KF = according to Karl Fischer)

The amorphous form of the salt was indicated by the X-ray powder diffraction pattern and DSC (plain halo effect and glass transition at about 40°C).

As an alternative, the esomeprazole arginine salt was obtained from the above solution by spray drying.

### Example 2 - Preparation of esomeprazole (5-methoxy-2-[(S)-[(4-methoxy-3,5-dimethyl-2-pyridinyl)methyl]sulphinyl]-1H-benzimidazole

a) 40.0 g (0.0974 mole) of 5-methoxy-2-[(S)-[(4-methoxy-3,5-dimethyl-2-pyridinyl)methyl] sulphinyl]-1*H*-benzimidazole magnesium salt trihydrate were dissolved in 400 ml of methanol at 15-20°C. The pH value of the solution was adjusted to 8.0 with 6% acetic acid and the solution was diluted with 200 ml of water. The esomeprazole was extracted with methylene chloride and the extracts were treated with activated charcoal. After concentrating the extracts, 225 ml of water were added and the pH value was adjusted to 12.0 with 20% sodium hydroxide. The layers were separated. 50 ml of methanol were added to the aqueous layer, and then the product was precipitated with 25 % acetic acid at 15 - 20°C and filtered. The wet precipitate was re-slurried in alkaline water for one hour, filtered and dried under reduced pressure at room temperature and subsequently at 30-35° for 5 hours. 20.5 g of amorphous form of esomeprazole were obtained and characterized by the following analyses:
   ¹H-NMR (300 MHz, DMSO-d₆): 2.17 (s, 3H, CH₃), 2.19 (s, 3H, CH₃), 3.68 (s, 3H, OCH₃), 3.79 (s, 3H, OCH₃), 4.64 (d, J_{HA,B}= 13.4Hz, 1H, H_{A}), 4.76 (d, J_{HA,B}= 13.4Hz,1H, H_{B}), 6.88 (dd, J_{H6,7}=8.8Hz, J_{H4.7}=2.4Hz, 1H, H₇), 7.08 (d, J_{H4,7}=2.4Hz, 1H, H₄), 7.52 (d, J_{H6,7}=8.8Hz, 1H, H₆), 8.18 (s, 1H, H₆,)
   HPLC (Chiral purity) = 100.0%
   Moisture content (KF) = 0.89%
b) 6.0 g (0.017 mole) of esomeprazole were dissolved in 60 ml of methanol and then this solution was added to 108 ml of alkaline water having a pH of above 7. The solution was clarified by filtration and the product was isolated by lyophilization. The obtained freeze dried product was further dried under reduced pressure at room temperature and then at 30-35° for 3 hours. The amorphous salt was produced with 100 % yield and characterized by the following analyses:
   HPLC (Chiral purity) = 100.0%
   Moisture content (KF) = 1.03%

### Example 3

| Enteric coated pellets / SUSPENSION LAYERING | |
|---|---|
| **Ingredients** | **Composition** |
| **Core material** | |
| Esomeprazole arginine salt | 41.1 % |
| Microcrystalline cellulose pellets | 32.4% |
| Hydroxypropyl methylcellulose | 4.6 % |
| Magnesium carbonate | 21.2 % |
| Sodium lauryl sulphate | 0.7 |
| | |

| **Separating layer** | |
|---|---|
| Core material | 79.5 % |
| Hydroxypropyl methylcellulose | 8.7 % |
| Talc | 7.9 % |
| Dibasic sodium phosphate | 1.7 % |
| Magnesium stearate | 2.2 % |
| | |

| **Enteric coating** | |
|---|---|
| Pellets with separating layer | 62.7 % |
| Methacrylic acid ethyl acrylate copolymer | 31.2 % |
| Triethyl citrate | 3.7 % |
| Glyceryl monostearate | 1.9 % |
| Polysorbate 80 | 0.5 % |
| | |

| **Overcoating** | |
|---|---|
| Pellets with enteric coating | 91.95 % |
| Hydroxypropyl methylcellulose | 4.5 % |
| Microcrystalline cellulose | 2.5 % |
| Polyethylene glycol 6000 | 0.3 % |
| Titanium dioxide | 0.75 % |
| | |

| **Tablets** | |
|---|---|
| Pellets with overcoating | 29.8 % |
| Microcrystalline cellulose | 70 % |
| Sodium stearyl fumarate | 0.2 % |

A drug suspension was prepared using esomeprazole arginine salt, hydroxypropyl methylcellulose, magnesium carbonate, sodium lauryl sulphate and purified water. Suspension layering of sugar spheres was performed in a fluid bed processor using bottom spray technique (Wurster column). The spray operation was stopped when the specified amount of bulk liquid had been sprayed.

The prepared core material was dried until the loss on drying of the pellets was about 1-1.5 % and covered with a separating layer in a Wurster column with a hydroxypropyl methylcellulose solution containing talc, dibasic sodium phosphate and magnesium stearate.

The enteric coating suspension was prepared using methacrylic acid ethyl acrylate copolymer (Eudragit L 30 D-55), triethyl citrate, glyceryl monostearate and Polysorbate 80. The suspension was sprayed onto the pellets covered with separating layer in a fluid bed apparatus. The spray operation was stopped when the specified amount of bulk liquid had been sprayed, and then drying was carried in the Wurster column.

Additional coating was applied in the Wurster column by spraying the dispersion of hydroxypropyl methylcellulose, microcrystalline cellulose, polyethylene glycol and titanium dioxide on the enteric-coated pellets. Drying in the same apparatus followed the procedure.

The resulting pellets were classified by sieving. Microcrystalline cellulose and sodium stearyl fumarate were added and the mixture was compressed into tablets using a rotary tableting machine.

### Example 4

| Enteric coated pellets / EXTRUSION & SPHERONIZATION | |
|---|---|
| **Ingredients** | **Composition** |
| **Core material** | |
| Esomeprazole arginine salt | 28 % |
| Mannitol | 44.7 % |
| Hydroxypropyl cellulose | 4.5 % |
| Microcrystalline cellulose | 12.2 % |
| Magnesium carbonate | 10.2 % |
| Sodium lauryl sulphate | 0.4 % |
| | |

| **Separating layer** | |
|---|---|
| Core material | 88.9 % |
| Hydroxypropyl methylcellulose | 8.7 % |
| Magnesium carbonate | 2.4 % |
| | |

| **Enteric coating** | |
|---|---|
| Pellets with separating layer | 60.3 % |
| Methacrylic acid ethyl acrylate copolymer | 31 % |
| Ethyl acrylate methyl methacrylate copolymer | 3 % |
| Propylenglycole | 4 % |
| Glyceryl monostearate | 1.5 % |
| Polysorbate 80 | 0.2 % |
| | |

| **Overcoating** | |
|---|---|
| Pellets with enteric coating | 92 % |
| Hydroxypropyl methylcellulose | 3.5 % |
| Microcrystalline cellulose | 3.5 % |
| Polyethylene glycol 6000 | 1 % |
| | |

| **Tablets** | |
|---|---|
| Pellets with overcoating | 40 % |
| Microcrystalline cellulose | 59.8 % |
| Sodium stearyl fumarate | 0.2 % |

Esomeprazole arginine, mannitol, hydroxypropyl cellulose, microcrystalline cellulose and magnesium carbonate were premixed in a mixer. By adding an aqueous solution of sodium lauryl sulphate the mass was wet-mixed to a proper consistency. The wet mass was pressed through an extruder and spheronized to pellets. The pellets were dried in a fluid bed dryer until the loss on drying was about 1-1.5 %, and then they were classified by sieving.

The prepared core material was covered with a separating layer. Magnesium carbonate was suspended in a hydroxypropyl methylcellulose solution and the dispersion was sprayed onto agitated pellets in a Wurster column.

The enteric coating layer was applied to the pellets covered with separating layer using an aqueous dispersion of methacrylic acid ethyl acrylate copolymer (Eudragit L 30 D-55) and ethyl acrylate methyl methacrylate copolymer (Eudragit NE 30 D) plasticized with propylenglycole to which a glyceryl monostearate dispersion had been added. The suspension was sprayed onto the pellets covered with the separating layer in a Wurster column until the specified amount of bulk liquid had been sprayed. The drying process was carried out in the same equipment.

Additional coating was applied in a Wurster column by spraying a dispersion of hydroxypropyl methylcellulose, microcrystalline cellulose and polyethylene glycol on the enteric-coated pellets.

The resulting pellets were dried, classified by sieving and compressed into tablets together with microcrystalline cellulose and sodium stearyl fumarate using a rotary tableting machine.

### Example 5

| Enteric coated tablets | |
|---|---|
| **Ingredients** | **Composition** |
| **Tablet core** | |
| Esomeprazole arginine salt | 10.8 % |
| Lactose | 61.6 % |
| Hydroxypropyl cellulose | 2.8 % |
| Hydroxypropyl cellulose (low substitution) | 3.9 % |
| Talc | 2.7 % |
| L-arginine | 3.6 % |
| Magnesium stearate | 1 % |
| | |

| **Separating layer** | |
|---|---|
| Hydroxypropyl methylcellulose | 1.9 % |
| Talc | 0.9 % |
| Magnesium hydroxide | 0.7 % |
| | |

| **Enteric coating layer** | |
|---|---|
| Hydroxypropyl methylcellulose phthalate | 7.1 % |
| Talc | 1.1 % |
| Titanium dioxide | 1 % |
| Diethyl phthalate | 0.9 % |

The following powders were mixed together: lactose, esomeprazole arginine salt, hydroxypropyl cellulose (low degree of substitution), talc and L-arginine. Hydroxypropyl cellulose was dissolved in a sufficient quantity of purified water and used as granulation liquid for the powder mixture. After the granulation, the granules were dried, sieved, magnesium stearate was added and tablets were prepared by compressing the granules using a rotary compression machine.

The tablet cores were further coated with a separating layer. The isopropyl alcohol/methylene chloride dispersion of hydroxypropyl methylcellulose, magnesium hydroxide and talc was sprayed onto the tablets in a suitable coating pan until a desired weight gain was reached. The drying was carried out in the coating pan.

Such coated tablets were loaded in a perforated coating pan to be further coated with an enteric layer. An enteric coating suspension was prepared by dispersing hydroxypropyl methylcellulose phthalate, talc, titanium dioxide and diethyl phthalate in the mixture of methanol and methylene chloride. The enteric coating suspension was sprayed simultaneously. The spraying operation was stopped when the specified amount of liquid had been sprayed, and then drying was carried out in the coating pan.

## Claims

1. Esomeprazole arginine salt.

2. Salt according to claim 1, wherein the molar ratio between esomeprazole and arginine is about 1:1.

3. Salt according to claim 1 or 2, which is in amorphous form.

4. Process for preparing the salt according to any one of claims 1 to 3, wherein esomeprazole and arginine are reacted in a solvent and the solvent is removed.

5. Process according to claim 4, wherein the solvent is selected from water, an alcohol or a mixture thereof.

6. Process according to claim 5, wherein the alcohol is selected from methanol, ethanol, 2-propanol, n-propanol and butanol.

7. Process according to claim 5 or 6, which is carried out until all esomeprazole is dissolved or the pH value of the reaction mixture remains constant.

8. Pharmaceutical composition comprising the salt according to any one of claims 1 to 3.

9. Composition according to claim 8, which is in form of pellets or tablets.

10. Composition according to claim 9, wherein the pellets comprise
(a) a core including the salt according to any one of claims 1 to 3,
(b) optionally at least one separating layer,
(c) at least one enteric layer, and
(d) optionally at least one over-coating layer.

11. Composition according to claim 9, wherein the tablets comprise
(a) a core including the salt according to any one of claims 1 to 3,
(b) optionally at least one separating layer, and
(c) at least one enteric layer.

12. Composition according to claim 10 or 11, wherein the salt is included in the core in form of a layer covering the core.

13. Multiple-unit tablets which are obtainable by compressing a mixture of the pellets according to claim 9 or 10 with tablet excipients.
